# EUROPEAN PATENT APPLICATION

(11) **EP 1 229 129 A2**
(43) Date of publication of application: **07.08.2002**
(21) Application number: 01131009.1
(22) Date of filing: 28.12.2001
(51) Int. Cl.: C12Q 1/68, G01N 21/00

(54) **Affinity detecting/analytical chip, method for production thereof, detection method and detection system using same**

(30) Priority: 28.12.2000 JP 2000402451
(71) Applicant: EBARA CORPORATION, Ohta-ku, Tokyo (JP)
(72) Inventor: Nagasawa, Hiroshi, Hirakata-shi, Osaka (JP)
(74) Representative: Wagner, Karl H., Dipl.-Ing.

(57) **Abstract**

An affinity detecting/analytical chip comprises a plurality of capillaries bundled together, the capillaries having, fixed to their inner surface, probe molecules causing different specific binding reactions. A detection method flows molecules as a sample into the bundled capillaries to cause specific binding reactions, thereby binding the sample molecules to the inner surface of the capillaries, then applies light from one end of the capillary bundle, and observes light exiting from the opposite end surface of the capillary bundle, thereby examining the capillaries for the presence of binding. A reaction product detection system uses the chip. The chip, the detection method, and the detection system secure a sufficient signal strength, permit easy detection with a daylight lamp, and reduce difficulty involved in fluorescence detection.

## Description

This invention relates to an affinity detecting/analytical chip, which is used for genetic diagnosis and physiological function diagnosis and enables recognition of many functional molecules, a method for its production, and a detection system using it.

An affinity detection method, which uses a substance selectively binding to a particular molecule, and selectively detects a corresponding substance, is a very sensitive method of detection. It has been used, for example, in liquid chromatography as an affinity column for detecting a particular protein with the use of a particular enzyme. However, this affinity detection method in liquid chromatography gives information only on a particular molecule, and is not analytical means simultaneously presenting information on the existence of many molecules.

Currently, detection of polymorphism due to variation of a gene, especially variation of one base (sequence), for example, is effective for diagnosis of a disease ascribed to mutation or the like, e.g., cancer, and is also necessary for guidelines on drug response and adverse reactions, contributing to analysis of genes related to causes of multiple factor diseases and to predictive medical care. The use of a so-called DNA chip, a type of the affinity detection method, is known to be effective for this detection. A hitherto utilized DNA chip having short DNA strands fixed thereto, so-called Gene Chip of Affymetrix, comprises 10,000 or more oligo-DNA fragments (DNA probes) fabricated, usually, on a silicon or glass substrate about 1 cm square by photolithography technology. When a fluorescence labeled DNA sample to be examined is flowed on this DNA chip, DNA fragments having sequences complementary to the probes on the DNA chip bind to the probes. Only the bound portions can be distinguished by fluorescence, and the particular sequences of the DNA fragments in the DNA sample can be recognized and quantitatively determined. This method has already been shown to be capable of detecting mutation of an oncogene or detecting gene polymorphism.

A microarray having cDNA's arranged on a slide glass is also used.

These chip-shaped affinity detection methods are very effective as means of analysis for simultaneously offering information on the existence of many molecules. However, partly because of small detection areas available, sufficient signal strengths are not ensured. For this and other reasons, light absorption analysis as used in liquid chromatography or the like is not usable, and high sensitivity analysis relying on fluorescence detection is the only method used. This method is applicable only to analysis in a particular region.

Thus, what is called proteome analysis has been made only by means such as two-dimensional electrophoresis.

Under these circumstances, a demand is growing for means of detection and analysis which uses a substance selectively binding to a particular molecule and selectively detects a corresponding substance, simultaneously provides information on the existence of many molecules, and permits high sensitivity analysis without labor.

The object of the present invention is to establish a method for preparing a reaction probe chip which ensures a sufficient signal strength, permits easy detection by the ultraviolet-visible absorption method, and decreases difficulty involved in fluorescence detection, and to provide an affinity detecting/analytical chip and a detection system which can be used in various diagnoses of physiological functions, including DNA polymorphism.

To attain the foregoing object, the present inventors conducted various studies on materials for and shapes of reaction probe chips in an attempt to realize a reaction probe chip which has on the surface a high integration degree comparable to that obtained by the aforementioned photolithography facilities, without using photolithographic technologies requiring a lengthy, complicated reaction process, posing difficulty in flexibly attaining different objects, and involving huge costs, and without using an expensive high sensitivity analytic device for fluorescence detection.

Based on these studies, the inventors produced a resin molded detection chip comprising a bundle of different capillaries precisely positioned and bound together, each of the capillaries having probe molecules fixed to an inner surface thereof. They noticed that the above-described problems could be solved by introducing a sample into the resin molded detection chip. This finding led them to accomplish the present invention.

The present invention solves the above problems by the following means:
(1) An affinity detecting/analytical chip comprising a plurality of capillaries bundled together, the capillaries having, fixed to an inner surface thereof, probe molecules causing different specific binding reactions.
(2) The chip of (1), wherein the probe molecules are DNA's, RNA's or PNA's and fragments thereof, oligonucleotides having arbitrary base sequences, antigens, antibodies or epitopes, and enzymes, proteins or functional site polypeptide chains thereof.
(3) A method for producing the chip of (1) or (2), comprising bundling different capillaries by use of suitable binding means while precisely positioning the capillaries, the capillaries having the probe molecules fixed to or synthesized on the inner surface thereof beforehand.
(4) A detection method comprising:
   flowing molecules as a sample into a bundled of capillaries having, fixed to an inner surface thereof, probe molecules causing different specific binding reactions, to cause specific binding reactions, thereby binding the sample molecules to the inner surface of the capillaries;
   then applying light from one end of the capillary bundle; and
   observing light exiting from an opposite end surface of the capillary bundle, thereby examining the capillaries for presence of binding.
(5) A reaction product detection system comprising:
   a binding device for binding an analyte to an affinity detecting/analytical chip comprising a plurality of capillaries precisely positioned, bundled together by binding means, and further resin molded, each of the capillaries having probe molecules fixed to an inner surface thereof;
   a light absorption observation device comprising a module housing portion for accommodating the analyte-bound affinity detecting/analytical chip, a light emitting portion provided ahead of the module housing portion, and an observation unit provided behind the module housing portion; and
   a data processing device connected to the light absorption observation device.

The key factors in the present invention are the establishment of a light absorption analytic method which holds an analyte, applied to genetic diagnosis and physiological function diagnosis, by an affinity carrier being a capillary widely used in gas chromatography, electrophoresis or liquid chromatography, and which also uses the capillary as a multiple reflection light absorption cell for detection to increase signal strength; and the bundling of many of the capillaries, thereby achieving high sensitivity, simultaneous multiple detection means having the same function as performed by the simultaneous operation of many affinity capillary liquid chromatographic columns.

According to the present invention, there can be easily provided means of detection and analysis which uses a reactive probe substance, such as a protein having an arbitrary configuration or an oligonucleotide having an arbitrary base sequence, namely, a substance selectively binding to a particular molecule, and selectively detects a substance reactive with this substance, simultaneously gives information on the existence of many molecules, and permits high sensitivity analysis without labor and without requiring special equipment such as photolithography equipment.

In the present invention, moreover, if capillaries bearing various reactive substances are ready for use, an affinity detecting/analytical chip having various types of reactive substance probes fixed thereto can be supplied more conveniently by a necessary combination on a necessary occasion. As noted here, the present invention can provide a lower cost, higher stability affinity detecting/analytical chip. Hence, the preparation of an affinity detecting/analytical chip for DNA or the like, which meets individuals' needs, becomes possible, contributing to made-to-order medical care.

Furthermore, the present invention ensures higher sensitivity detection free from burden on the sample, for example, no need for matching of the sample with a fluorescence indicator. Also, various pigments can be used at the same time. Thus, simultaneous multi-type analysis becomes feasible. Unlike the existing DNA chips, the chip of the present invention provides means for detection in a new field, such as protein detection.

The present invention will become more fully understood from the detailed description given hereinbelow and the accompanying drawings which are given by way of illustration only, and thus are not limitative of the present invention, and wherein:
FIG. 1 is an overall image of glass capillaries bundled at the center of an affinity detecting/analytical chip;
FIG. 2 is a perspective view for explaining the concept of the capillary having probe molecules fixed inside;
FIG. 3 is a conceptual view showing the fixation of probe molecule cDNA's into the capillary;
FIG. 4 is a conceptual view showing the surface synthesis of probe molecule oligo-DNA's inside the capillary;
FIG. 5 is a conceptual perspective view showing an example of a process for bundling the capillaries;
FIG. 6 is a perspective view showing an example of a resin mold for fixing the bundled capillaries;
FIG. 7 is an explanation drawing of an operation for affinity binding of a sample to be analyzed to a detecting/analytical chip, a resin molded capillary bundle;
FIG. 8 is a perspective view for explaining affinity binding of the sample to the interior of the capillary;
FIG. 9 is an conceptual explanation drawing of a method for detecting the capillaries that reacted;
FIG. 10 is an conceptual explanation drawing of the principle of measurement of affinity detection/analysis;
FIG. 11 is an explanation drawing of a sectional pattern during observation in affinity detection/analysis; and
FIG. 12 is a schematic explanation drawing of devices/system for affinity detection/analysis.

First, the probe molecule will be described briefly.

When a DNA probe is used, for example, RNA coding for albumin can be analyzed by a technique called Northern blotting. Also, hybridization can be performed by Southern blotting to use the hybrids for analysis of DNA.

Furthermore, DNA hybridization using a synthetic oligonucleotide having an arbitrary base sequence as a labeled probe makes it possible to detect a corresponding sequence from the genome.

The method of detecting a particular base sequence, which exists in situ in a living organism, by use of a nucleic acid probe is used on DNA in the chromosome and on RNA in the cell. Chemical labeling of the DNA probe is known to improve resolution greatly.

Next, embodiments of the present invention will be described in more detail with reference to the drawings. [Structure and method for preparation of affinity detecting/analytical chip]

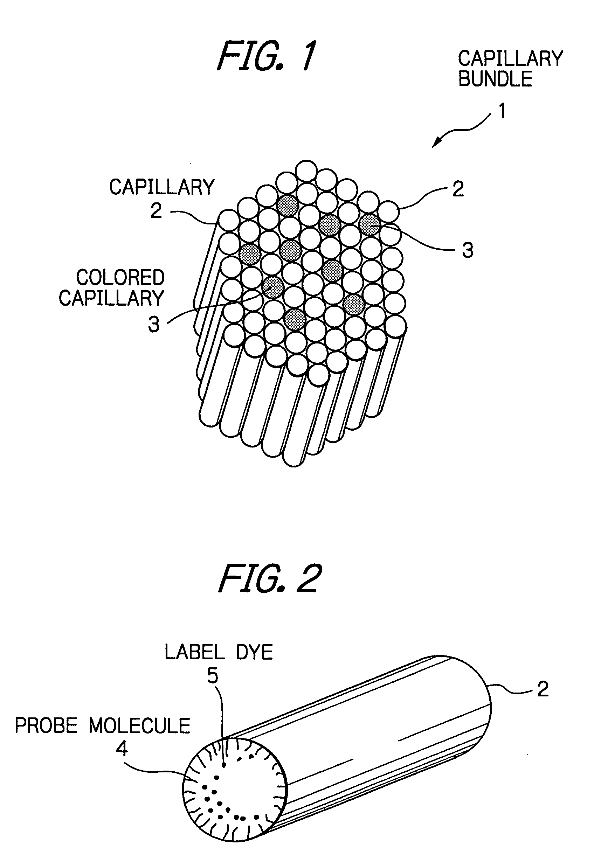

FIG. 1 shows an image of a central portion of an affinity detecting/analytical chip. Of bundled capillaries, the capillaries reacted with an assay object having absorption in the visible region look colored. In FIG. 1, the numeral 1 denotes a bundle of glass capillaries, in which colored capillaries 3 reacted with the assay object are scattered among many capillaries 2.

The individual capillaries 2 constituting the capillary bundle 1 are preferably composed of a material, such as glass or quartz glass, and preferably have an internal diameter of about 10 to 100 microns, but may be of a larger size. The glass as the raw material may be of any grade, but preferably is quartz glass, and is also preferably boro-silicate glass. Any material, which gives the same structure, may be used for the capillary, and it is not limited to a vitreous material, but may be an organic or plastic material.

The inner surface of the capillary must initially have reactivity, and is preferably silanol groups. The length of the capillary is not prescribed, but is preferably about 1 to 5 mm. From the viewpoint of the principle of detection to be described later, the longer the capillary, the higher sensitivity is obtained for analysis, but the lower the operating efficiency is. Thus, an appropriate length and an appropriate thickness should be selected.

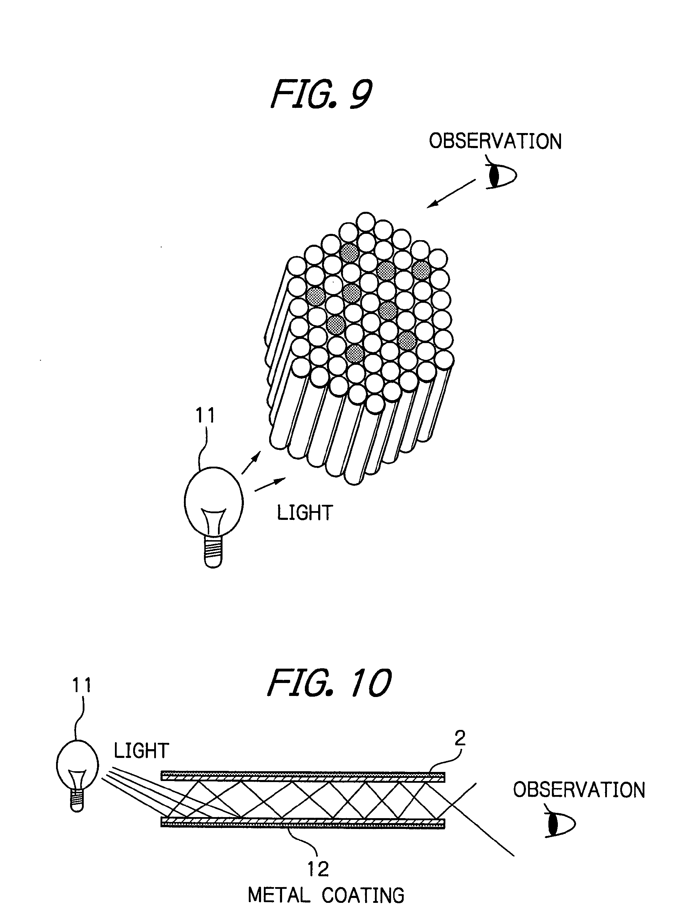

If the capillary is made of quartz glass, it is necessary to compensate for the brittleness of quartz glass and impart elasticity to quartz glass. For this purpose, it is preferred to apply an outer coating with polyimide or the like. Alternatively, the outside of the capillary is preferably coated with metal by, for example, vapor deposition. This is to facilitate amplification of a very pale coloration by frequently repeating reflection within the capillary, as will be explained later with reference to FIG. 10.

Conventionally, metals have been considered unsuitable as materials for the capillary, but the progress of metal surface treatment techniques has put metals to uses as stainless capillaries or metal capillaries more inert, tougher and usable at higher temperatures than quartz glass. Preferred examples of the plastic materials are polyetheretherketone, polyethylene, and polypropylene, and even polytetrafluoroethylene can be used if treated on the inner surface.

In the case of these materials, their diameters, lengths, and types may be selected according to the purpose of detection.

FIG. 2 is a conceptual view of the capillary 2 having probe molecules 4 fixed inside. Molecules usable as the probe molecules 4 may be any molecules showing affinity. For example, there can be used DNA's, RNA's or PNA's (peptide nucleic acids) and their fragments, oligonucleotides having arbitrary base sequences, antigens, antibodies or epitopes, and enzymes, proteins or their functional site polypeptide chains.

The numeral 5 denotes a label dye. As the label, a fluorescent substance is often used, but an isotope can also be used. In this case, characteristic advantages can be obtained.

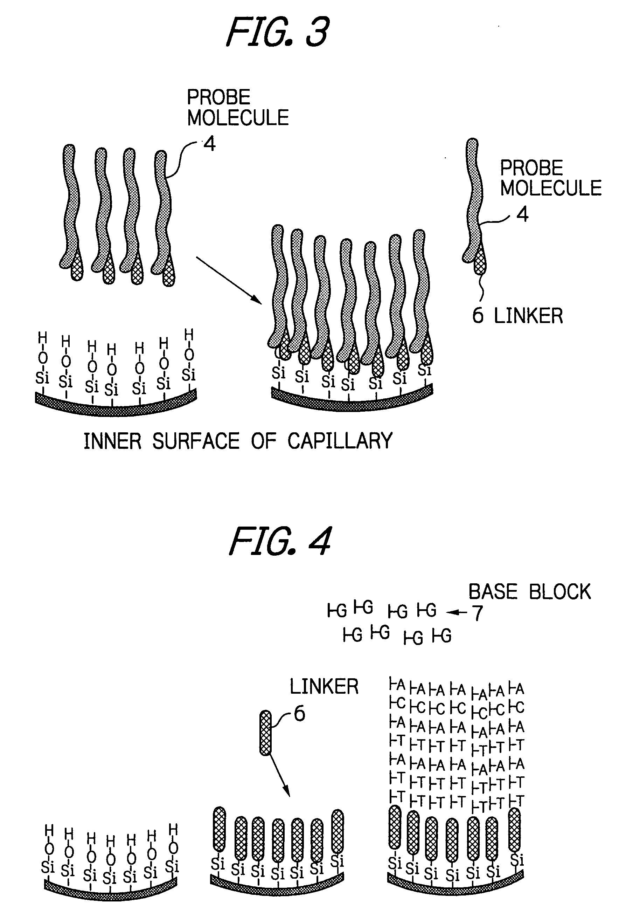

FIGS. 3 and 4 are views showing how to fix these probe molecules 4. When a probe known as cDNA (complementary DNA) is to be fixed, for example, a linker 6 may be used to fix the probe inside the capillary 2.

At this time, the capillary bundle 1, made up of many individual capillaries 2, permits binding reactions to be effected at the same time in a reactor. This method is highly efficient, and these capillaries 2 maintain the same quality. Thus, the amounts of the probes 4 borne, and so on are easy to manage by sampling inspection.

If an oligonucleotide is used as the probe molecule 4, a base sequence may be synthesized inside the capillary, for example, by use of a method known as the phosphoamidite method. In this case, many individual capillaries 2 can be put together, and binding reactions can be simultaneously performed in the reactor. Thus, this procedure is highly efficient. Furthermore, these capillaries 2 maintain the same quality. Thus, the amounts of the probes borne, and so on are easy to manage by sampling inspection. Besides, cutting-off and purification are unnecessary, so that the capillary can be produced at a low cost.

In addition, synthesis takes place in a relatively large hole, and thus large oligonucleotide probes of 100 bases or more can be prepared with ease.

FIG. 3 is a process chart showing the fixing of cDNA's as the probe molecules 4 to the interior of the capillary 2. FIG. 4 is a process chart showing the fixing of oligo-DNA's as the probe molecules 4 onto the inner surface of the capillary 2. Both drawings illustrate the progress of the reaction from the left side to the right side of the drawing. In FIG. 3, the numeral 7 denotes a base block.

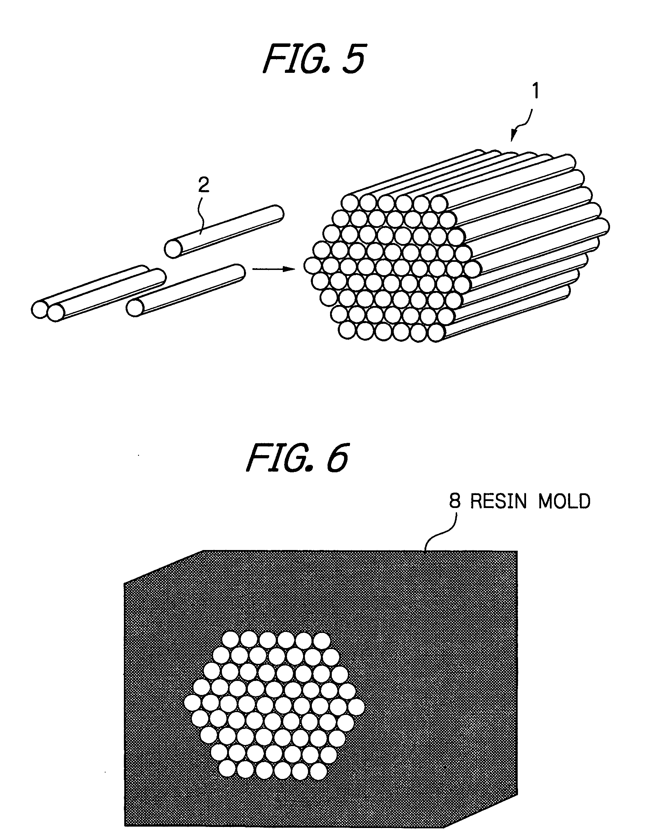

FIG. 5 is a conceptual view showing a process for bundling these various capillaries 2. The capillaries are mechanically combined and arranged into a large bundle, but it has to be clarified which positions the capillaries having which probe molecules 4 fixed thereto are located at.

In this process, as shown in FIG. 6, resin or the like, which does not affect detection, may be used for molding (sealing up) the capillaries, as indicated by the numeral 8. In this case, the mold is preferably provided in an opaque color, preferably, a black color. To give this structure, short capillaries may be bundled, or long capillaries may be bundled, and then cut to form this structure.

Various methods may be adopted to bundle the capillaries 2. However, the addresses or locations of the capillaries are very important. Thus, it is preferred to employ an array system according to which their relative positions will not be displaced later.

The size of the bundled capillaries is very small. If 100 of the capillaries with an outer diameter of 500 microns are bundled, the detection portion measures about 5 mm by 5 mm. Even in a bundle of 10,000 of the capillaries with an outer diameter of 200 microns, the detection portion measures only about 40 mm × 40 mm. Large scale integration is possible in this manner.

### [Reaction]

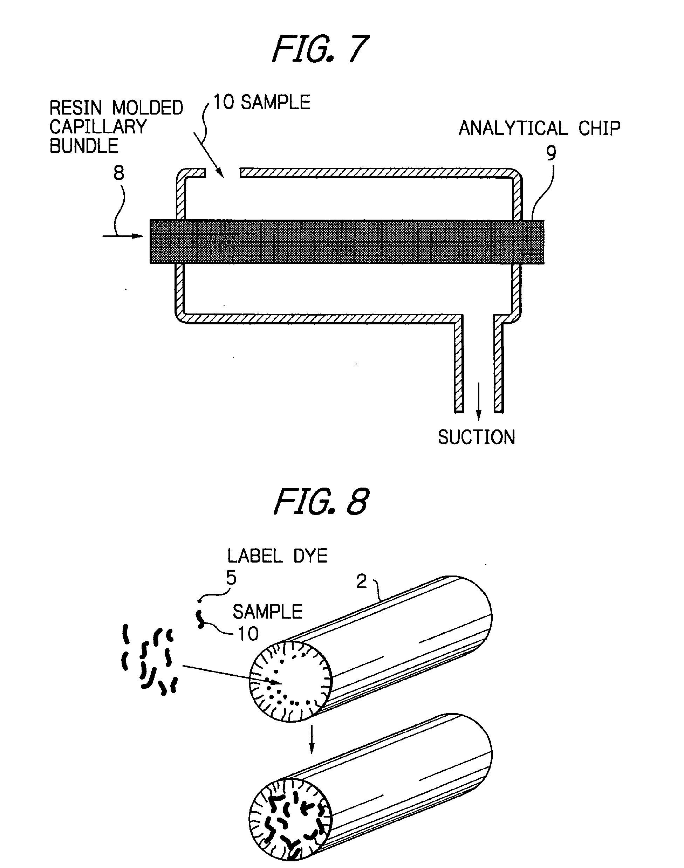

The reaction of a sample with the affinity detecting/analytical chip is performed by gripping a chip 9 from above and from below, and flowing a sample 10, to be analyzed, uniformly into the capillaries 2, as shown in FIG. 7. Dipping or any other method may be used, if it ensures such flowing.

During this procedure, the sample 10 flows within the capillaries 2, and is held within the capillaries 2 in the presence of specific reactions, if any. The sample 10 being flowed, if it has no absorption, may be stained with a label dye 5, or may be labeled with a fluorescent agent in some cases. By so doing, the interior of the capillary 2 is covered with a material having absorption, depending on the presence of reactivity.

### [Detection]

FIG. 9 conceptually shows a method for detecting the capillaries 3 that reacted. The bundle of the capillaries illuminated by a W₁D₂ lamp 11 from back shows differences between colored capillaries and uncolored capillaries as light goes out.

FIG. 10 shows the principle behind this phenomenon. Reflection is repeated many times within the capillary 2, whereby a very pale coloration is amplified and observed as a deep coloration. Thus, an analyzed value with very high sensitivity is obtained. Provision of a metal coating 12 on the outside of the capillary 2 is advantageous, because reflectivity increases.

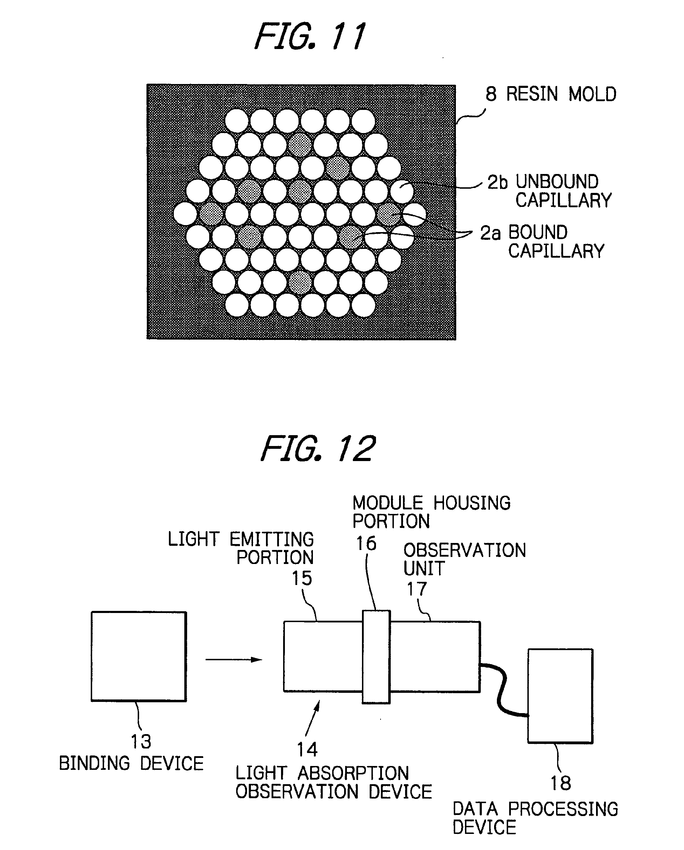

FIG. 11 shows a pattern actually obtained. Since the surroundings are an opaque resin mold 8, differences in color tone are clearly visible. The numeral 2a denotes bound capillaries, and the numeral 2b denotes an unbound capillary.

In the present invention, as described above, the label dye 5 easy to detect can be used. If the sample 10 itself has an absorption, detection is easier, so that difficulty involved in detection of fluorescence can be reduced. In detecting a fluorescence, irradiation with laser light is carried out from the detection side. Thus, the quantity of light of the fluorescence obtained is so small that detection is difficult. Moreover, an irradiation device used for laser light application is expensive. On the other hand, an irradiation device using the W₁D₂ lamp 11 is very inexpensive, and this is also of great advantage.

In this case as well, it goes without saying that fluorescence detection is possible with the present invention.

### [System and devices]

To implement the above-described detection system, a dedicated reaction binding device 13 is preferably available, and a certain type of light absorption detection system for reading the reacted chip 9 is necessary.

FIG. 12 is a conceptual view of devices used for this system. The analytical chip is disposed in the binding device 13. A solution containing the molecules 10 to be detected is flowed into the capillary bundle 1 of the analytical chip to cause specific binding reactions, thereby binding the sample molecules to the inner surface of the capillaries 2.

According to this detection system and devices, the chip 9, reacted in the binding device 13, is robotically transported into a light absorption observation device 14. The light absorption observation device 14 applies light to the chip 9 from one end, and observes light exiting from the opposite end surface, thereby examining the capillaries for the presence of binding. In FIG. 12, a light emitting portion 15, a module housing portion, and an observation unit 17 are integrally combined to constitute the light absorption observation device 14. The numeral 18 denotes a processing device for data from the observation unit 17.

The present invention will be described in further detail by reference to the following examples, which in no way limit the invention.

### [Example 1]

The inner surface of a quartz glass capillary having an internal diameter of 13 microns and a length of 5 mm was treated by amination. Then, cDNA's obtained from various libraries were bound to the interior of the capillaries via glutaraldehyde as a linker. A bundle was formed from 100 of the capillaries by use of an arraying machine, and molded with epoxy resin to obtain an affinity detecting/analytical chip.

A dye-labeled cDNA as a sample was flowed through the chip, and then the chip was dried. The chip was illuminated with a D₂ lamp from behind, and UV-detected. Particular capillaries were found to show absorption.

### [Example 2]

The inner surface of a Pyrex glass capillary having an internal diameter of 20 microns and a length of 10 mm was treated by amination. Then, various oligonucleotides of 50 bases were synthesized in the capillaries by the phosphoamidite method using succinic acid as a linker. A bundle was formed from 100 of the capillaries by use of an arraying machine, and molded with epoxy resin. The resin molded bundle was sliced onto a 1 mm thick plate to obtain an affinity detecting/analytical chip.

Dye-labeled cDNA as a sample was flowed through the chip, and then the chip was dried. When the chip was illuminated with a W (tungsten) lamp from behind, particular capillaries were found to develop a color.

While the present invention has been described in the foregoing fashion, it is to be understood that the invention is not limited thereby, but may be varied in many other ways. Such variations are not to be regarded as a departure from the spirit and scope of the invention, and all such modifications as would be obvious to one skilled in the art are intended to be included within the scope of the appended claims.

## Claims

1. An affinity detecting/analytical chip comprising a plurality of capillaries bundled together, said capillaries having, fixed to an inner surface thereof, probe molecules causing different specific binding reactions.

2. The chip of claim 1, wherein the probe molecules are DNA's, RNA's or PNA's and fragments thereof, oligonucleotides having arbitrary base sequences, antigens, antibodies or epitopes, and enzymes, proteins or functional site polypeptide chains thereof.

3. A method for producing the chip of claim 1 or 2, comprising bundling different capillaries by use of suitable binding means while precisely positioning the capillaries, said capillaries having the probe molecules fixed to or synthesized on the inner surface thereof beforehand.

4. A detection method comprising:
flowing molecules as a sample into a bundled of capillaries having, fixed to an inner surface thereof, probe molecules causing different specific binding reactions, to cause specific binding reactions, thereby binding the sample molecules to the inner surface of the capillaries;
then applying light from one end of the capillary bundle; and
observing light exiting from an opposite end surface of the capillary bundle, thereby examining the capillaries for presence of binding.

5. A reaction product detection system comprising:
a binding device for binding an analyte to an affinity detecting/analytical chip comprising a plurality of capillaries precisely positioned, bundled together by binding means, and further resin molded, each of said capillaries having probe molecules fixed to an inner surface thereof;
a light absorption observation device comprising a module housing portion for accommodating the analyte-bound affinity detecting/analytical chip, a light emitting portion provided ahead of the module housing portion, and an observation unit provided behind the module housing portion; and
a data processing device connected to the light absorption observation device.
